Europäisches Patentamt

European Patent Office

Office européen des brevets

(19)

(11) Publication number: **0 270 699 B1**

## EUROPEAN PATENT SPECIFICATION

(12)

(45) Date of publication of patent specification: **01.07.92**

(51) Int. Cl.⁵: **A61H 23/04**, A61H 1/00

(21) Application number: **86117044.7**

(22) Date of filing: **08.12.86**

(54) **An air-massage chair.**

(43) Date of publication of application:
**15.06.88 Bulletin 88/24**

(45) Publication of the grant of the patent:
**01.07.92 Bulletin 92/27**

(84) Designated Contracting States:
**DE FR GB SE**

(56) References cited:
EP-A- 0 026 799          DE-B- 1 925 522
US-A- 3 297 023          US-A- 3 595 223
US-A- 3 613 671          US-A- 4 186 734

(73) Proprietor: **Hara, Katsumasa
No. 3-8-19, Kamishakujii
Nerima-ku, Tokyo(JP)**

(72) Inventor: **Hara, Katsumasa
No. 3-8-19, Kamishakujii
Nerima-ku, Tokyo(JP)**

(74) Representative: **Schwabe - Sandmair - Marx
Stuntzstrasse 16
W-8000 München 80(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to an air-massage chair which provides a massage action with an air pressure and a backbone straightening action with an adequate flexing movement for a human body at its relaxing portions when sitting on the chair, thereby to improve the massage effect and which may be readily fabricated and assembled.

Generally, a sitting position on a chair seems a most relaxing condition for a human body in a daily life. Further, there have been various proposals for preventing fatigue of the body because of the sitting time being elongated recently. In general, various psychological and physical stress and fatigue are mainly concentrated on a back area along a backbone of a human body. Such stress and fatigue are considerably relaxed when sitting on a chair, because a most load is transferred to a hip area thereby to eliminate the load from the back area. When the back area of the body is contacted slightly against the back of the chair, the load transfer to the hip area may be alleviated. When sitting on the chair, the hip area is stably fixed while other body areas, such as upper and lower body portions may be flexibly and comfortably moved in any direction. Thus, the continued sitting position cannot cause the stress and fatigue on the back area of the body, so that the considerable psychological and physical relaxation may be achieved compared with, for example, relaxation when lying on a bed.

In view of the foregoing, there have hitherto been proposed and commercialized various massage devices and finger-press devices, in which a portion of the human body, especially a backbone is placed against the back area of the chair for pressing, vibrating, massaging or hitting either side of the backbone, or in which a mechanically operating means for vertical rolling action is arranged.

In such type of the massage- or finger-press devices, however, the restriction of the forcible local pressure to either side of the backbone may neglect a physiological condition of the human body. As a result, a stronger stimulus may be applied to a muscle, a ligament and a backbone thereby to aggravate blood circulation. Thus, neither relaxation of the psychological and physical stress and fatigue, nor the massage- and straightening effects with the flexing movement can be expected. Consequently, such devices are unsuitable for massaging old-aged, diseased or handicapped person with rigid muscle and ligament or with a deteriorated bone.

US-PS 4,186,734 discloses an inflatable seat unit having plurality of adjacent, individual elongated air cells located on a seat which is, among other features, characterized by that said unit comprises a piece of foam material containing a cavity, and said air cells being completely located in said cavity. The inflatable part of the seat unit comprises the seat area and may also comprise the back area. In particular, the back area has no curve physiologically adapted to a curve of a vertebral column. Any hint with respect to the form of the back area is missing in said document.

Further, an air-massage device is known, in which a sheet composed of a plurality of air-tight air bags is wound around the body or extended on a mattress, and a compressed air is introduced into each air bag for its expansion and exhaused for its shrinkage in order to give an air pressure onto certain portions of the body, thereby to achieve an effective massage. Such type of air-massage device, however, necessitates the winding of the sheet with the air bags or the lying on the sheet, so that the human body can not be adequately relaxed and the medical or rehabilitation effect cannot be expected. Moreover, this type of air-massage device is troublesome in handling and thus inconvenient.

The inventor has now succeeded in developing an impressed air-massage device, in which a chair may fix and retain any body proportion, improve a massage function without deteriorating a human technological function of relaxing the human body, give a wavy air pressure to leg, hip and back areas thereby to adapt wide area of the body to the physiological condition without pressure or stress feeling, and provide a mild stimulus without side effects during its use for a long period of time.

The invention concerns an air-massage chair having a seat area and a back area on which a plurality of elongated air bags each laterally extending over substantially the whole width of the seat and back areas are wholly or partially arranged in a longitudinal direction of the seat and back areas, wherein the back area has a curve physiologically adapted to a curve of a vertebral column; a plurality of elevated areas each laterally extending across substantially the whole width of the seat and back area are provided in a longitudinal direction of the seat and back areas on the surfaces of at least the back area; and each of the elongated air bags is arranged on the respective elevated areas forming the base for the respective air bag.

In other words, the improved massage device is an air-massage chair comprising a plurality of air-tight elongated air bags arranged wholly or partially from the seat area upto the back area of the chair, which may reduce a pressure feeling to a human body and adapt to physiological conditions of any portion of the body from legs through a hip area upto a back area regardless of shape, height, weight, proportion and diseased portions of the body. Further, it may provide not only a massage effect but also a rehabilitation effect, as well as a medical treatment effect, such as a backbone straightening effect with adequate flexing movement.

In such air-massage chair, the form of the chair and the arrangement of the air tight air bags should be properly designed depending on the body proportion and the condition of the body, as well as the movement and the structure of the backbone. Further, in order to change body sites to be massaged depending on purposes of the massage, an extent of expansion and shrinkage of each unit of the air bags should be varied depending on the body sites. In this case, the air-tight air bags, which have previously been varied in their extent of the expansion and the shrinkage, are desirably combined.

Generally, a vertebra is in the shape of a relatively slight S-shaped curve from the neck through the breast to the waist. For this reason, adaptation of a massage portion or a dynamic portion to the S-shaped curve is desired for the physiological condition of the human body. Accordingly, unsuitable dynamic change for the physiological condition may apply an undesirable pressure onto a portion of the vertebral column and thus adversely affect the human body.

Accordingly, an object of the invention is to provide an air-massage chair, in which a seat area and a back area of the chair over their surfaces and substantially across their width are provided wholly or partially with a plurality of elongated air bags in parallel, and which may reduce a pressure feeling to a human body and adapt to physiological condition of any portion of the body from legs through the hip area upto the back area regardless of shape, height, weight, proportion and diseased portions of the body. Further, it may provide not only a massage effect but also a rehabilitation effect, as well as a medical treatment effect, such as a backbone straightening effect with adequate flexing movement.

Another object of the invention is to provide an air-massage chair, in which a frame body of the chair is constructed independently from air-massage bases having air-tight air bags of different shapes and constructions and is removably combined thereto depending on the body proportion of the patient and/or aims of the massage for enabling these aims to be conveniently achieved with convenient replacement of the air-tight air bags in case of their deterioration and damage, thereby to permit efficient and economical usage for a long period of time.

A further object of the invention is to provide an air-massage chair, in which air-tight air bags are arranged in such a manner that a back area of the chair is adapted to a normal vertebral shape of the human body for preventing a forcible pressure against the vertebral column, and in which expansion of the air bags is aligned in a specified direction for better adaption to a physiological condition of the human body, thereby to provide not only a massage effect but also a rehabilitation effect, as well as a medical treatment effect, such as a backbone straightening effect with adequate flexing movement.

The invention concerns an air-massage chair as claimed in claim 1. Preferred embodiments are set out in claims 2 to 7.

The invention will now be illustrated hereinbelow in more detail for its preferred embodiments with reference to the accompanying drawings.

Figure 1 shows a schematic construction of one embodiment of the air-massage chair acccording to the invention, which is explained also with regard to Figure 15, which is a perspective view of the basic construction of the air-massage chair of the invention;

Figure 2 shows a schematic construction of a variation of the air-massage chair as shown in Fig. 1;

Figure 3 shows a schematic construction of another air-massage chair according to the invention;

Figure 4 shows a schematic construction of a further embodiment of the air-massage chair according to the invention;

Figures 5a, 5b and 5c each show a schematic construction of an embodiment having an electrode in the air-massage chair according to the invention;

Figure 6 shows a schematic construction of a still further embodiment of the air-massage chair according to the invention;

Figure 7 is a perspective view of various elements in the air-massage chair according to one embodiment of the invention;

Figure 8 is a side view of the air-massage chair as shown in Fig. 7 in its assembled state for use;

Figures 9 to 12 are schematic views showing variation of an air-massage base useful in the air-massage chair as shown in Fig. 7;

Figures 13 and 14 are schematic views showing variation of combination of a frame base with an air-massage base in the air-massage chair as shown in Fig. 7;

Figure 15 is a perspective view of a basic construction of the air-massage chair according to the invention;

Figure 16 is a schematic view showing a preferred embodiment of the air-massage chair as shown in Fig. 15;

Figure 17 is a schematic view of main portions showing relation between the vertebra and the air-massage chair as shown in Fig. I6;

Figures I8a and I8b are sectional and perspective views, respectively, showing mounted state of air bags;

Figures I9a and I9b through 2Ia and 2Ib are sectional views of main portions of air bags in their shrinking and expanding states showing relation of different heights between the air bags and elevated areas attached thereto;

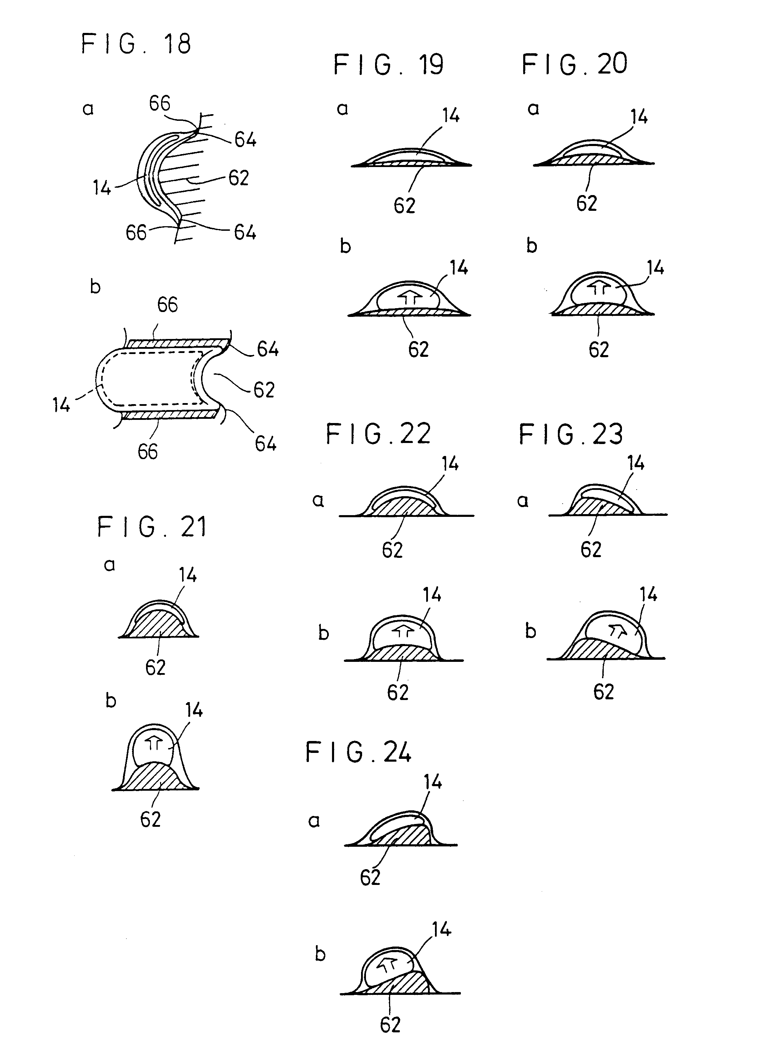

Figures 22a and 22b through 24a and 24b are sectional views of main portions of air bags in their shrinking and expanding states showing relation of orientation between the air bags and the elevated area; and projections attached thereto;

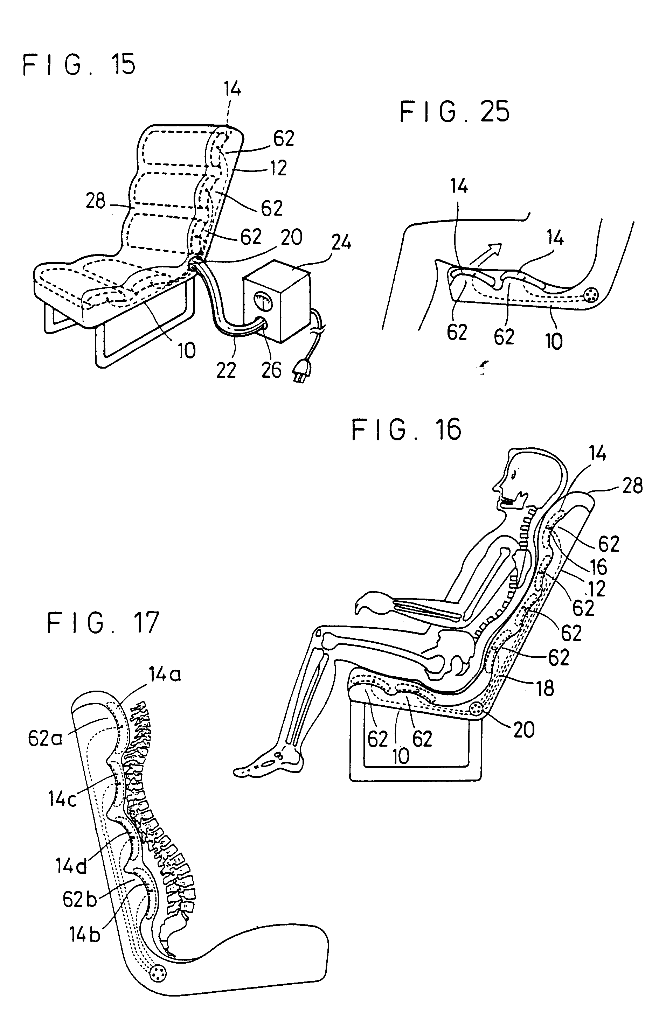

Figure 25 shows a mounted state of the air bags to a seat area of the chair.

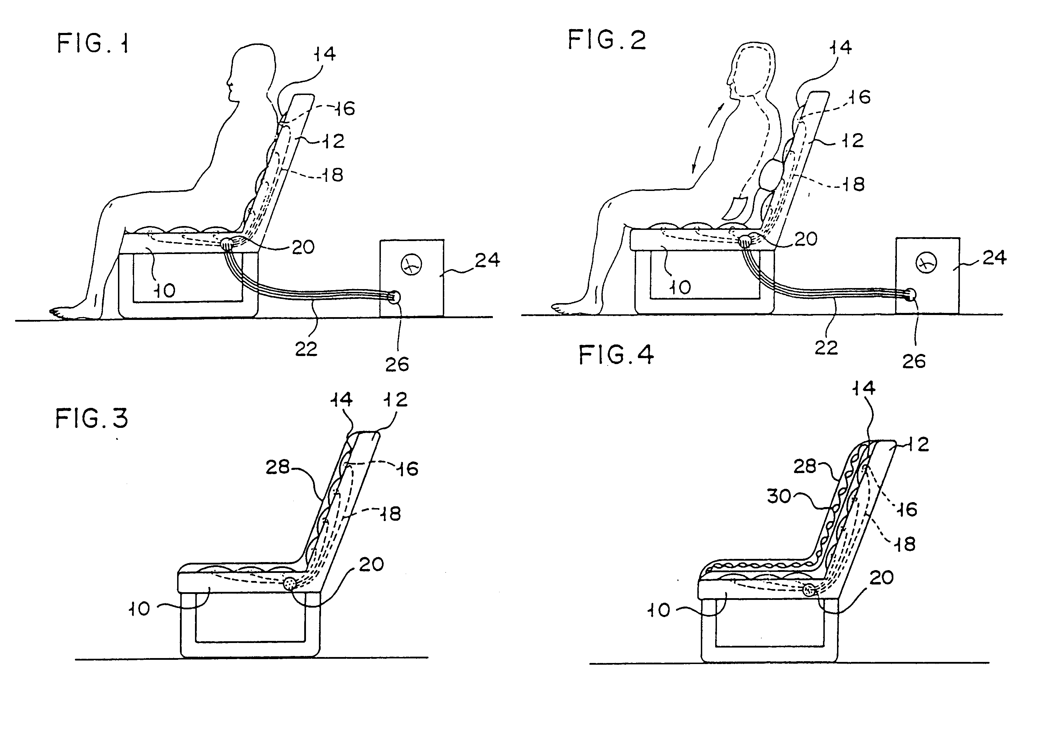

Figure I illustrates the most basic construction of the air-massage chair according to the invention, which comprises a horizontal seat area I0 and a back area I2 slightly inclined relative to the seat area I0, which areas may be covered with a cushion material for imparting a suitable cushioning property to their surfaces. The seat and back areas I0 and I2 of the chair are provided over their surfaces and substantially across their width wholly or partially with a plurality of elongated air bags I4 in parallel. Each air bag I4 is formed flat with an air-tight material having a good isolating property and is provided at its suitable portion with an air-feeding and exhausting port I6 which is connected to an air tube I8. A plurality of the air tubes I8 are arranged within the chair and combined at a connector section 20 on one side of the chair. A combined tube 22 is then connected to a connector section 26 of an air controller 24. Figure 15 is a perspective view showing the air-massage chair according to the invention, the principle of which is identical to the embodiment of Fig. 1, except that the seat and back areas 10, 12 their surfaces are provided substantially across the entire width of the seat and back areas 10, 12 with a plurality of elevated areas 62 each being made of a thick cushion material for retaining a suitable cushioning property. Each of the elevated areas 62 on the seat and back areas 10, 12 is provided with the elongated air bag 14. The back area 12 of the chair according to Figure 15 has a curve physiologically adapted to the curve of a human vertebral column. The combination of the air bag 14 with the air controller 24 is identical to that in Fig. 1.

When sitting on the air-massage chair thus constructed, as shown in Fig. 16, and starting operation of the air controller 24, the same effective massage and straightening effects as in the previous embodiments may be obtained.

Especially in this chair, the arrangement of the air bag 14 on each elevated area 62 enables the height and orientation of the area 62 to be varied upon expansion of the air bag 14, so that each portion of the human body may be subjected to the physiologically suitable massage effect.

For example, Figure 17 shows a normal curve of a vertebra in a general healthy person. As apparent from the curve, when a back of the human body is subjected to the wavy pressure applied by the air bags 14 of the air-massage chair according to the invention, the pressure should correspond to the curve. As illustrated, for example, the elevated area 62a and 62b corresponding to a cervical vertebra and a lumbar vertebra may be somewhat more elevated than other portions in consideration of the physiological curve or higher expandable air bags 14a and 14b may be arranged, while less expandable air bags 14c and 14d are arranged corresponding to a thoracic vertebra. Thus, the effective massage may be achieved depending on the different portions of the human body and the physiological condition.

Figures 18a and 18b show a mounted state of the air bag 14 to the elevated area 62. The air bag 14 at its either edge 66, 66 is fixed along respective opposite raised portion 64, 64 of the elevated area 62 by means of an adhesive or welding.

Figures 19a, 19b through 21a, 21b show different stages of expansion of the air bag 14 due to different heights of the elevated area 62, wherein Figure 19 corresponds to the state of lower height difference of the area 62 in which a cross-section of the air bag 14 on its expansion shows a gentle hill, while Figure 20 corresponds to the state of medium height difference of the area 62 in which a cross-section of the air bag 14 on its expansion shows a mountain shape. Figure 21 corresponds to the state of higher height difference of the area 62 in which a cross-section of the air bag 14 on its expansion shows a steep slope of mountain. Thus, the air bag 14 may vary its degree of pressure depending on the different stages.

Figures 22a, 22b through 24a, 24b show different stages of the air bag 14 on its expansion due to orientation of the elevated area 62, wherein Figure 22 corresponds to a symmetric shape of the area 62 in which the air bag expands vertically relative to a horizontal base plane of the area 62. Figure 23 corresponds to a deviated state of the area 62 toward the left in which the air bag 14 expands inclined toward the right relative to the horizontal base plane of the area 62, while Figure 24 corresponds to a deviated state of the area 62 toward the right in which the air bag 14 expands inclined toward the left relative to the horizontal base plane of the area 62. Thus, the air bag 14 may vary its pressure orientation due to the expanding direction.

As described above, combination of the height difference with the orientation of the elevated area 62 may achieve a number of variations in the pressure pattern against the human body on expansion of the air bag 14. As a result, the pressure of the air bag 14 suitable to the physiological condition may be readily achieved.

Figure 25 illustrates a construction of the elevated area 62 and the air bag 14 on the seat area 10, which enables the air bag 14 to expand from a thigh toward a hip of the body.

When sitting on the air-massage chair thus constructed and starting operation of the air controller 24, the plural aligned air bags 14 are rapidly fed with an compressed air or exhausted to expand or shrink sequentially, thereby to provide a wavy pressure of a good feeling for the contacted areas of the human body, as well as an adequate flexing movement. Thus, the massage effect of promoting circulation of blood and lymphatic fluid suitable for the physiological condition of the body, as well as the backbone straightening effect for relaxing the vertebral column, muscle and ligament together with the adequate flexing movement may be achieved.

Figure 2 illustrates a variation of the air-massage chair, as previously described, in which the air bags 14 on the back area 12 of the chair are protruded semi-spherically in cross-section when expanded by the compressed air. Thus, the expanded air bags 14 may provide the good pressure feeling and the local pressure onto the back area of the body, thereby to give proper flexing movement to the vertebral column, as well as to shoulder-blades, ribs and pelvis, to relax the surrounding muscle, ligament and nerves and to promote the circulation of blood and lymphatic fluid, resulting in the excellent straightening effect with adequate flexing movement more than the massage effect. Other remaining construction of this embodiment is similar to that of Fig. 1, so that the same reference may be used for the same elements for omitting the detailed description therefor.

Although the previous embodiment has been described with a functional construction of the air bags 14, the latter may covered with a cover 28 of a strechable material or a larger size for absorbing expansion of the air bags in order to improve an appearance of the chair, as shown in Fig. 3. Alternatively, as shown in Fig. 4, an elastic sheet 30 having a plurality of elastic projections may be preferably utilized instead of or together with the cover 28.

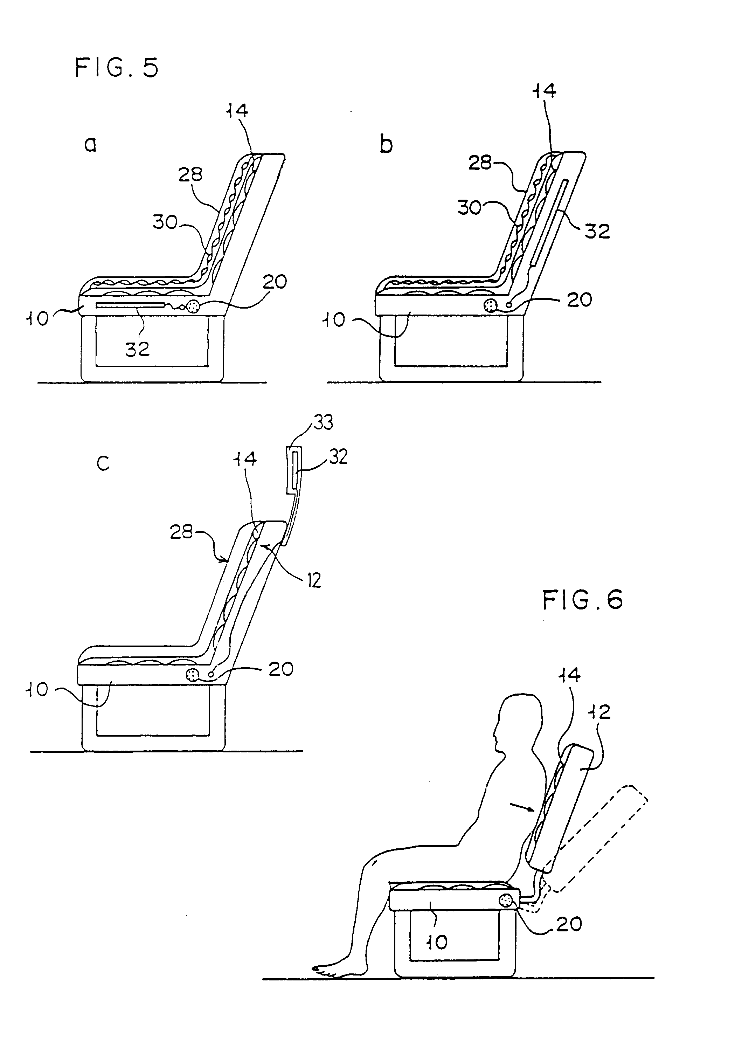

Figures 5a to 5c illustrate another embodiment of the air-massage chair according to the invention, in which the chair according to the invention at its seat area 10 (Fig. 5a) or back area 12 (Fig. 5b) contains therein an electrode 32 as a medical treatment device of electric field. In order to arrange the electrode 32 at the back area 12, there may be additionally provided an isolating support 33 into which the electrode 32 may be incorporated (Fig. 5c). Thus, the medical treatment function of electric field may be added to the air-massage chair for improving its function readily and conveniently.

Figure 6 illustrates a modification of the air-massage chair according to the invention, in which the back area 12 is resiliently or stepwise inclinable relative to the seat area 10 in order to vary the function of the chair per se. Thus, the user may incline the chair at any angle depending on his desire to achieve more comfortable massage effect.

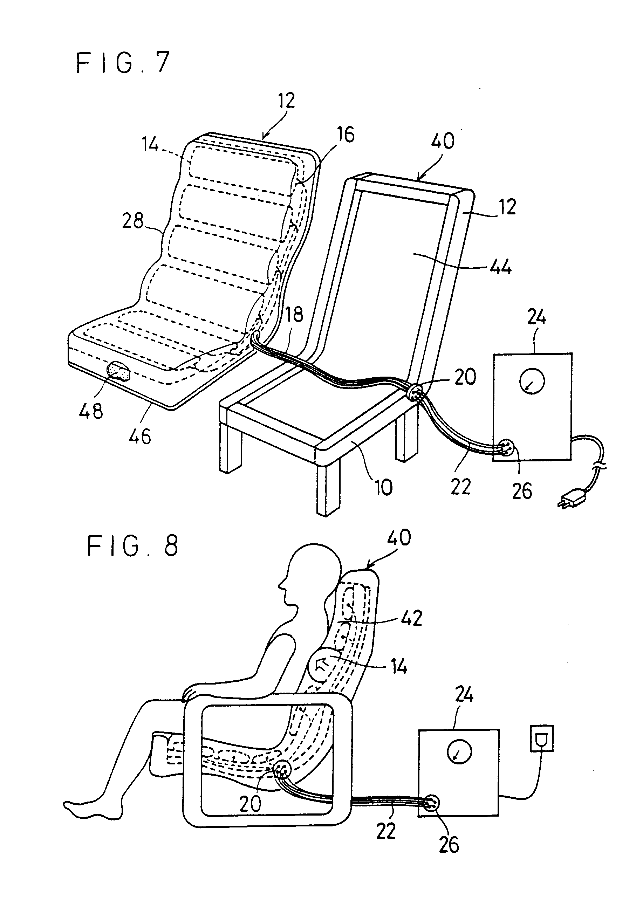

Figure 7 illustrates a basic construction of a further embodiment of the air-massage chair according to the invention, which comprises a frame body 40 and an air-massage base 42. The frame body 40 includes a seat area 10 and a back area 12 which are designed for their size and angle in consideration of a human body technology in order to achieve most relaxed physiological condition when sitting thereon. The frame body 40 from the seat area to the back area 12 is provided with a recess 44 for mounting the air-massage base 42. The base 42, on the other hand, is provided with a rigid base plate 46 to be fitted into the recess 44 of the frame body 40. Over the rigid base plate 46 is extended a cushion material 48 of a suitable thickness, on which a plurality of elongated air bags 14 secured wholly or partially across the entire width of the cushion. The combination of the air bags 14 with the air controller 24 is identical to that in Fig. 1.

The air-massage chair thus constructed may be conveniently assembled by fitting the air-massage base 42 into the recess 44 of the frame body 40. When sitting on the assembled air-massage chair, as shown in Fig. 8, and starting operation of the air controller 24, the plural aligned air bags 14 are rapidly fed with a compressed air or exhausted to expand or shrink sequentially, thereby to provide a wavy pressure of a good feeling for the contacted area of the human body, as well as an adequate flexing movement. Thus, the massage effect of promoting circulation of blood and lymphatic fluid suitable for the physiological condition of the body, as well as the backbone straightening effect for relaxing the vertebral column, muscle and ligament together with the adequate flexing movement may be achieved.

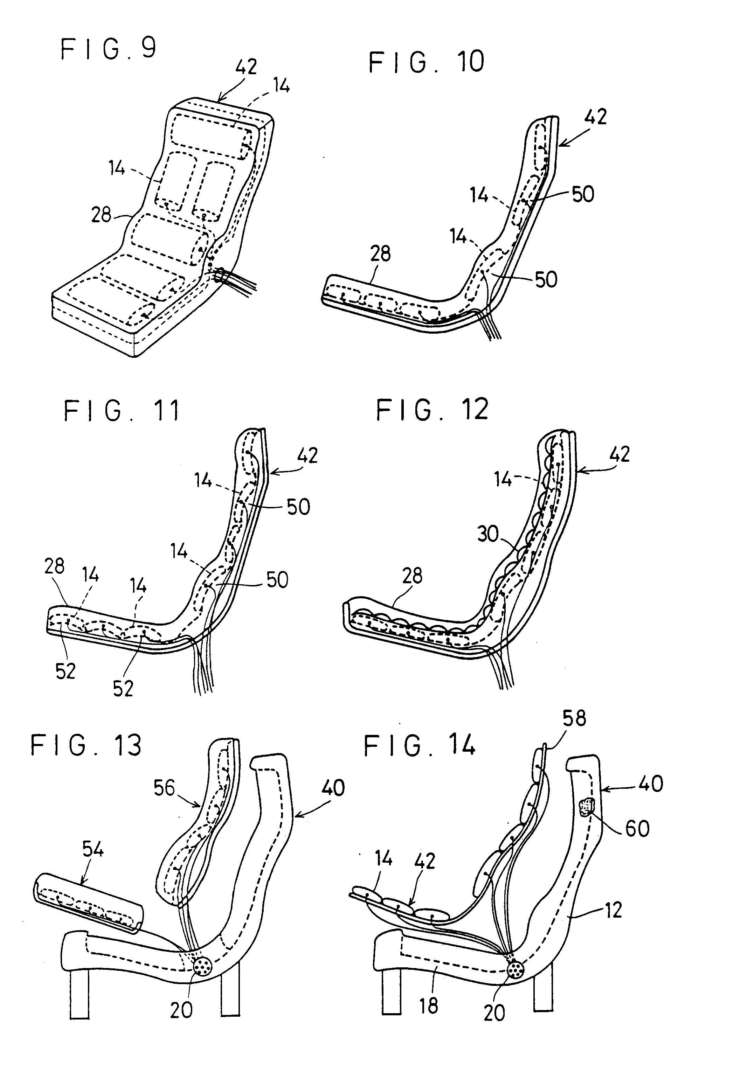

Figures 9 to 12 illustrate a variation of the air-massage base 42, as shown in Fig. 7. In Fig. 9, a pair of air bags 14a and 14b selected from the air bags 14 on the back area 12 are longitudinally arranged in parallel for achieving variation in the massage effect. In Fig. 10, the cushion material 48 on the back area across its entire width is provided with a plurality of partially elevated portions 50 over which the air bag l4 is secured for achieving variation of the massage effect. In Fig. ll, the partially elevated portions 52 are arranged also on the seat area across its entire width and covered with the air bag l4. In Fig. l2, the air bags l4 are covered with an elastic sheet 30 having elastic protrusions on its upper surface in order to achieve variation of the massage effect.

Figures l3 and l4 illustrate a modification of the combination of the frame body 40 with the air-massage base 42. In Fig. l3, the air-massage base 42 is divided into two parts which are arranged on the seat area 54 and the back area 56 and fitted into the recess of the frame body 40, respectively. In Fig. l4, in place of the rigid base plate of the air-massage base 42 there is employed an elastic base plate 58 over which is arranged the air bag l4 directly, while the frame body 40 on its recess side is provided a cushion material 60 of a required thickness over which is arranged the elastic base plate 58 for obtaining the similar massage effect.

As apparent from the previous description of the chair of the invention, the soft air-massage of the wavy and highly variable air pressure may be provided over the wide range of the human body around its back in the most relaxed state of the body, so that the excellent massage and backbone straightening effects with the adequate flexing movement may be obtained, which effects are most suitable for the physiological condition of the human body. Further, the mild pressure action without violent stimulus is effective and useful to the old-aged, diseased or handicapped person as the massage means. Furthermore, the high degree of air bag expansion, the combination with the elastic sheet, the inclination of the back area and the variation of the elevated areas may provide not only the massage effect but also the rehabilitation effect, while the incorporation of the electrode may produce the function of the medical treatment means of electric field.

In accordance with the invention, the frame base of the chair and the air-massage bases are independently constructed, so that their manufacture is very convenient and a number of variations may be made, for example, a variety of the frame bases may be combined with a variety of the air-massage bases on a mass production scale at a low cost depending on the aims and the applications. Moreover, repair and replacement of the air-massage base become very convenient upon its accident and damage and thus may contribute to its long service life considerably.

In the air-massage chair according to the invention, the arrangement of the elevated area capable of changing height difference and orientation on the seat and back areas of the chair may achieve variation of the pressure action on various portions of the human body, resulting in a variety of massage effects, as well as medical treatment effects suitable for the physiological condition of the human body.

**Claims**

1. An air-massage chair having a seat area (10) and a back area (12) on which a plurality of elongated air bags (14) each laterally extending over substantially the whole width of the seat and back areas (10, 12) are wholly or partially arranged in a longitudinal direction of the seat and back areas (10, 12), characterised in that
the back area (12) has a curve physiologically adapted to a curve of a vertebral column;
a plurality of elevated areas (62) each laterally extending across substantially the whole width of the seat and back areas (10, 12) are provided in a longitudinal direction of the seat and back areas (10, 12) on the surfaces of the seat and the back area (12); and
each of the elongated air bags (14) is arranged on the respective elevated area (62) forming the base for the respective air bag (14).

2. An air-massage chair according to claim 1, wherein the elevated areas (62) have different heights.

3. An air-massage chair according to claim 1 or claim 2, wherein the elongated air bags (14) are varied in height and orientation upon expansion of the elongated air bags (14) depending upon variation of height and orientation of the elevated areas (62).

4. An air-massage chair according to any of claims 1 to 3, comprising a frame body (40) of a chair shape having a seat area (10) and a back area (12), and an air-massage base which includes a rigid base plate (46) of a shape adapted to the frame body (40), a cushion material (48) extended over the base plate (46) so as to be adapted to a body proportion from a thigh to a back of a human body, a plurality of elongated air bags (14) laterally extending over a substantially whole width of the cushion material (48) and being wholly or partially arranged in a longitudinal direction thereof, and a cover for wholly covering the air bags (14), said rigid base plate (46) being removably mounted to a fitting portion of the seat area (10) and the back area (12) of the frame body (40).

**5.** An air-massage chair according to claim 4, wherein the air-massage base is divided into two separate parts which are arranged on the seat area (10) and the back area (12) and the rigid base plate (46) of which is removably mounted to the fitting portion of the seat area (10) and the back area (12) of the frame body (40).

**6.** An air-massage chair according to claim 4 or claim 5, wherein the frame body (40) has a sufficient width for arranging a plurality of air-massage bases (42), said air-massage bases (42) being removably mounted to a plurality of fitting portions which are formed at the seat area (10) and the back area (12) of the frame body (40).

**7.** An air-massage chair according to any of claims 4 to 6, wherein the frame body (40) and/or the air-massage base (42) is provided therein with an electrode (32) for a medical device of electric field.

**Revendications**

**1.** Fauteuil de massage pneumatique comportant une zone formant siège (10) et une zone formant dossier (12), sur lesquelles une pluralité de coussins gonflables allongés (14), qui s'étendent chacun latéralement sensiblement sur toute la largeur des zones (10,12) du siège et du dossier, sont disposés en totalité ou en partie dans une direction longitudinale des zones (10,12) formant siège et dossier, caractérisé en ce que la zone formant dossier (12) possède un profil adapté physiologiquement à un profil de la colonne vertébrale,
une pluralité de zones surélevées (62), dont chacune s'étend latéralement sensiblement sur toute la largeur des zones (10,12) formant siège et dossier, sont prévues dans une direction longitudinale des zones (10,12) formant siège et dossier, sur les surfaces de la zone formant siège et de la zone formant dossier (12); et chacun des coussins pneumatiques allongés (14) est disposé sur des zones surélevées respectives (62) formant la base pour le coussin pneumatique respectif (14).

**2.** Fauteuil de massage pneumatique selon la revendication 1, dans lequel les zones surélevées (62) possèdent des hauteurs différentes.

**3.** Fauteuil de massage pneumatique selon la revendication 1 ou 2, dans lequel la hauteur et l'orientation des coussins pneumatiques allongés (14) sont modifiées lors de l'expansion des coussins pneumatiques allongés (14), en fonction de la variation de la hauteur et de l'orientation des zones surélevées (62).

**4.** Fauteuil de massage pneumatique selon l'une quelconque des revendications 1 à 3, comprenant un corps formant cadre (40) ayant une forme de fauteuil comprenant une zone formant siège (10) et une zone formant dossier (12), et une base de massage pneumatique qui comprend une plaque de base rigide (46) possédant une forme adaptée au corps formant cadre (40), un matériau amortisseur (48) qui s'étend sur la plaque de base (46) de manière à être adapté à une partie du corps s'étendant depuis une cuisse jusqu'au dos d'un corps humain, une pluralité de coussins pneumatiques allongés (14) s'étendant latéralement sensiblement sur toute la largeur du matériau amortisseur (48) et disposés en totalité ou en partie dans une direction longitudinale de ce matériau, et un capot servant à recouvrir entièrement les coussins pneumatiques (14), ladite plaque de base rigide (46) étant montée de façon amovible à une partie de montage de la zone formant siège (10) et de la zone formant dossier (12) du corps formant cadre (40).

**5.** Fauteuil de massage pneumatique selon la revendication 4, dans lequel la base de massage pneumatique est subdivisée en deux parties séparées qui sont disposées sur la zone formant siège (10) et sur la zone formant dossier (12) et dont la plaque de base rigide (46) est montée de façon amovible sur la partie de montage de la zone formant siège (10) et de la zone formant dossier (12) du corps formant cadre (40).

**6.** Fauteuil de massage pneumatique selon la revendication 4 ou 5, dans lequel le corps formant cadre (40) possède une largeur suffisante pour la mise en place d'une pluralité de bases de massage pneumatique (42), lesdites bases de massage pneumatique (42) étant montées de façon amovible sur une pluralité de parties de montage qui sont formées dans la zone formant siège (10) et dans la zone formant dossier (12) du corps formant cadre (40).

**7.** Fauteuil de massage pneumatique selon l'une quelconque des revendications 4 à 6, dans lequel le corps formant cadre (40) et/ou la base de massage pneumatique (42) contiennent en eux une électrode (32) pour un dispositif médical d'application d'un champ électrique.

**Patentansprüche**

1.　Sitzmöbel für eine Massage mit Luft mit einem Sitzbereich (10) und einem Rückenbereich (12), an dem eine Vielzahl von länglichen Luftkissen (14), die sich jeweils seitlich über im wesentlichen die gesamte Breite des Sitzbereichs und des Rückenbereichs (10, 12) erstrecken, ganz oder teilweise in Längsrichtung des Sitzbereichs und Rückenbereichs (10, 12) angeordnet sind, dadurch gekennzeichnet daß
　　-　der Rückenbereich (12) eine Krümmung aufweist, die physiologisch der Krümmung einer Wirbelsäule angepaßt ist;
　　-　eine Vielzahl von erhöhten Bereichen (62), die sich jeweils seitlich über im wesentlichen die gesamte Breite des Sitzbereichs und Rückenbereichs (10, 12) erstrecken, in Längsrichtung des Sitzbereichs und Rückenbereichs (10, 12) auf den Oberflächen des Sitzbereichs und des Rückenbereichs (10, 12) vorgesehen ist; und
　　-　jedes der länglichen Luftkissen (14) auf dem jeweiligen erhöhten Bereich (62) angeordnet ist, der die Basis für das jeweilige Luftkissen (14) bildet.

2.　Sitzmöbel für eine Massage mit Luft nach Anspruch 1, worin die erhöhten Bereiche (62) verschiedene Höhen aufweisen.

3.　Sitzmöbel zur Massage mit Luft nach Anspruch 1 oder Anspruch 2, worin die länglichen Luftkissen (14) bei Ausdehnung der länglichen Luftkissen (14) in Abhängigkeit von einer Veränderung der Höhe und Orientierung der erhöhten Bereiche (62) in Höhe und Orientierung variiert werden.

4.　Sitzmöbel für eine Massage mit Luft nach irgendeinem der Ansprüche 1 bis 3, mit einem Rahmen-Körper (40) in Form eines Sitzmöbels mit einem Sitzbereich (10) und einem Rückenbereich (12) sowie einer Luftmassagen-Basis, welche eine starre Grundplatte (46) mit einer solchen Form, daß sie dem Rahmen-Körper (40) angepaßt ist, ein Polstermaterial (48), welches sich so über die Grundplatte (46) erstreckt, daß es den Körperproportionen vom Oberschenkel bis zum Rücken eines menschlichen Körpers angepaßt ist, eine Vielzahl von länglichen Luftkissen (14), die sich seitlich über im wesentlichen die gesamte Breite des Polstermaterials (48) erstrecken und ganz oder teilweise in Längsrichtung dazu angeordnet sind, und einen Überzug zum Bedecken der Luftkissen (14) insgesamt einschließt, wobei die starre Grundplatte (46) entfernbar an einem Befestigungs-Bereich des Sitzbereichs (10) und des Rückenbereichs (12) des Rahmen-Körpers (40) montiert ist.

5.　Sitzmöbel für eine Massage mit Luft nach Anspruch 4, worin die Luftmassagen-Basis in zwei getrennte Teile aufgeteilt ist, die auf dem Sitzbereich (10) und dem Rückenbereich (12) angeordnet sind, und deren starre Grundplatte (46) entfernbar an dem Befestigungs-Bereich (10) des Sitzbereiches und des Rückenbereichs (12) des Rahmen-Körpers (40) montiert ist.

6.　Sitzmöbel für eine Massage mit Luft nach Anspruch 4 oder Anspruch 5, worin der Rahmen-Körper (40) eine ausreichende Breite für das Anordnen einer Vielzahl von Luftmassagen-Basen (42) aufweist, wobei die Luftmassagen-Basen entfernbar an einer Vielzahl von Befestigungs-Bereichen montiert sind, die auf dem Sitzbereich (10) und dem Rückenbereich (12) des Rahmen-Körpers (40) ausgebildet sind.

7.　Sitzmöbel für eine Massage mit Luft nach irgendeinem der Ansprüche 4 bis 6, worin der Rahmen-Körper (40) und/oder die Luftmassagen-Basis (42) mit einer Elektrode (32) für eine medizinisch wirksame Vorrichtung für ein elektrisches Feld versehen ist.

FIG.1

FIG.2

FIG.3

FIG.4

EP 0 270 699 B1

FIG. 5

a

b

C

FIG. 6

FIG. 7

FIG. 8

FIG. 9

FIG. 10

FIG. 11

FIG. 12

FIG. 13

FIG. 14

FIG. 18

a

66
64
14
62
66
64

b

66
64
14
62
66
64

FIG. 21

a

14
62

b

14
62

FIG. 19

a

14
62

b

14
62

FIG. 22

a

14
62

b

14
62

FIG. 24

a

14
62

b

14
62

FIG. 20

a

14
62

b

14
62

FIG. 23

a

14
62

b

14
62

FIG. 15

FIG. 25

FIG. 16

FIG. 17